# EUROPEAN PATENT SPECIFICATION

(11) **EP 2 589 389 B1**
(45) Date of publication and mention of the grant of the patent: **07.03.2018**
(21) Application number: 11801207.9
(22) Date of filing: 13.05.2011
(51) Int. Cl.: A61K 35/30, A61K 35/54

(54) **METHOD FOR PRODUCING A BIOLOGICALLY ACTIVE COMPLEX. BIOLOGICALLY ACTIVE PROTEIN/POLYPEPTIDE COMPLEX**
VERFAHREN ZUR HERSTELLUNG EINES BIOLOGISCH AKTIVEN KOMPLEXES BIOLOGISCH AKTIVER PROTEIN-/POLYPEPTID-KOMPLEX
PROCÉDÉ DE PRODUCTION D'UN COMPLEXE BIOACTIF ET COMPLEXE PROTÉIQUE ET POLYPEPTIDIQUE BIOACTIF

(30) Priority: 01.07.2010 RU 2010126871
(43) Date of publication of application: 08.05.2013
(73) Proprietor: Aktsionernoe Obschestvo "Pharm-Sintez", Moskow, 111024 (RU)
(72) Inventor: NAZARENKO, Anna Borisovna, Moscow 121614 (RU); SOKOLOV, Mikhail Anatolevich, Moscow 107150 (RU)
(74) Representative: Held, Stephan
(86) International application number: PCT/RU2011/000326
(87) International publication number: WO 2012/002837

(56) References cited:
- WO-A1-2009/088314
- WO-A1-2010/007620
- WO-A1-2010/007620
- WO-A2-02/064748
- FR-A1- 2 599 972
- RU-C1- 2 049 472
- RU-C1- 2 104 702
- RU-C1- 2 128 511
- US-A- 4 067 963

## Description

### PERTINENT ART

The Invention is as defiend in the present claims relates to the biologically active complex production method and biologically active protein-polypeptide complex. The complex is a biologically active substance obtained from the hoof livestock brain embryonic tissue. It is intended for use in the pharmaceutical production of the drugs stimulating the nervous tissue physiologic and reparative regeneration; and for use in the production of the medicinal drugs to treat the central and peripheral nervous system diseases. The Invention relates to its production method, as well.

### PRIOR ART

Today, on the background of the implacable vascular, toxic, infectious, and autoimmune genesis nervous system diseases incidence growth, no drugs of the nervous system specific direct reparants group are available. The object of this Invention is to create a biologically active substance with tissue-specific reparative-regenerative effect on the nervous tissue by extracting a protein-polypeptide complex from the hoof livestock brain embryonic tissue.

The substance is a biologically active protein-polypeptide complex wherein the complex composing protein-polypeptide components molecular mass is within the 5 to 200 kDa range; the minimum 10 to 120 kDa range medium molecular weight faction share is 80%; and the total protein concentration is 0.8 to 4.2 mg/ml. The substance is obtained by the ion exchange chromatography of the tissue homogenate filtrate, in presence of the buffer solution, detergents, proteolysis inhibitors, and solubilizers, from the brain tissue of the gestation mid first third to mid last third hoof livestock embryos.

The claimed protein-polypeptide complex biologic and pharmacologic activity is due to the efficient concentration ratios of the water-soluble negatively charged faintly acid neutral proteins and polypeptides relating to the growth factor, differentiation factor, and signaling molecules, ensuring thus the tissue-specific reparative-regenerative effect.

A number of the animal feedstock nootropic and neurometabolic activity protein-peptide drugs used to treat the nervous system diseases is known. These include:
1. Cerebrolysin: drug to treat the hemorrhagic or ischemic strokes, by Ebewe (Austria) (Encyclopedia of Drugs, 2006, p. 970) [1], a product of the intact porcine brain treatment;
2. Cerebrocurin: drug to treat the diseases associated with the central nervous system dysfunctions, by OOO "NIR" (Ukraine) (RF Patent No 2128511(1999) [2], a product of the animal embryonic brain treatment;
3. Cortexin: polypeptide nature drug obtained by extraction from the bovine and porcine brain cortex, by OOO "Geropharm" (Russia) (RF Patent No 2104702 (1999) [3], it has the nootropic, cerebroprotective, anticonvulsant, and antioxidative effects;
4. Cerebrolysate: drug improving the brain tissue resistance to the intoxication, hypoxia, hypoglycemia, and mechanical injury, by "Microgen" scientific-production association, a federal state unitary enterprise (Immunopreparat) (RF Patent No 2049472 (1999) [4]; it has the nootropic effect, and is a bovine brain cortex hydrolysate.

The Cerebrolysin contains the amino acids (∼85%), low molecular weight peptides (-15%), and microelements; the feedstock is the porcine brain. The drug's neuroprotective and trophic effects are due to the specific peptides and amino acids, of max. 10000 Dalton molecular mass, where the alanine, leucine, and lysine dominate and determine the drug properties. The drug is intended for the intramuscular and intravenous administration; the biologically active components concentration is low, hence, the treatment is high dose and long term. The detriments of the known drug are the long term treatment, low activity and specificity due to the feeble neuroprotective effect; and extremely low regenerative and reparative potentials for the nervous tissue.

The Cerebrocurin contains the water-soluble prenatal neuropeptides and the products of cleavage of the inactive high molecular weight precursors of the animal embryos brain homogenate, this homogenate is, after dilution in physiological solution, exposed to the immobilized proteolytic enzyme; the interaction conditions are defined on the basis of the target preparation check sample; and the resulting solution undergoes a 30-day cure at max. 10°C. This technique offers a higher (compared with the Cerebrolysin) activity of the resulting drug and significantly expands its spectrum of action, due to the higher mass of the peptides and low molecular weight proteins remaining after proteolysis. However, despite the long time solution formation before the aggregation and proteolysis processes end, this technique does not yield the purity required for the intravenous use; hence, the lesser bioavailability. The aqueous extraction without the solution, detergents, proteolysis inhibitors, and solubilizers buffering at the Cerebrocurin production phases, and the undetermined animal brain gestation terms do not allow for the necessary standardization; hence, the significant discrepancies as of the evolutionarily fixed protein-peptide concentrations ratios, and lower tissue-specific neuroregenerative activity and pharmacologic effect stability pharmacologic, all being limited to the feedback type biologic effect due to the tissue proteolysis products signal function.

The Cortexin contains the complex of the water-soluble biologically active alkaline, acid, and neutral polypeptides, 500 to 15000 Da molecular mass, and 3.5 to 9.5 isoelectric point. These are obtained from the livestock brain blenderized frozen tissue; the process consists in extracting with the zinc chloride containing acetic acid solution, separating the sediment, treating the supernatant liquid with acetone, drying with further purifying, sterilizing, and freeze drying the target product.

The drug is intended for the intramuscular and intravenous administration, the biologically active low molecular weight components concentration is low; hence, the treatment is high dose and long term. Though the Cortexin participates in regulating the inhibitory and exciting amino acids ratios, and concentrating the serotonin and dopamine, has a GABA-positive effect, reduces the toxic effects of the neurotropic drugs, improves the learning and memory processes, accelerates the brain functions rehabilitation after stresses, its activity and specificity are not high due to the feeble neuroprotective effect, its regenerative potential is low and reparative potential is insignificant for the nervous tissue; hence, the long term protracted treatment.

The Cerebrolysate also contains the water-soluble polypeptides obtained in the bovine brain cortex hydrolysis; its enzymatic activity is feeble; its molecular mass is max. 15000 Da. The mentioned in the Patent variation of the drug amino acid composition admits a significant compositional variation of the resulting peptide concentration ratios, determining the low tissue-specific activity and pharmacologic effect stability with the feeble neurometabolic and unexpressed nootropic effects.

### DISCLOSURE OF INVENTION

The object of this application is to create the biologically active substance with extended properties and high biological activity, with tissue-specific reparative-regenerative effect on the nervous tissue, and to develop its production method by separating a biologically active protein-polypeptide complex from the hoof livestock brain embryonic tissue; the problem solution consists in the selected combination of actions and conditions. The most important was to define the optimal embryonic brain gestational ages, and to select the reagents, their doses, extraction, buffering and proteolytic activity regulation conditions, to preserve the efficient concentration ratios of the proteins and polypeptides, evolutionarily fixed and determining the substance biological activity, ensuring the reparative-regenerative tissue-specific effect.

The problem solution consists in complying with all the feedstock procurement parameters and characteristics, and with the claimed method for producing a biologically active protein polypeptide complex formulation comprising actions involving quick frozen embryonic brain of hoof livestock of gestational age from the middle of the first trimester to the middle of the last trimester of pregnancy freezing and gradual, stage-by-stage, defrosting, at temperatures from 2°C to 28°C;
- homogenization in buffer solution with simultaneous extraction in the presence of proteolysis inhibitors and non-ionic detergents, including ethylenediamine tetraacetate, mannitol or maltose, with pH not less than 5.2 and not exceeding 8.5, with the volumetric solution-tissue ratio of at least 1 : 0.5;
- the homogenate is separated from undiluted tissue and cellular components by filtration and subsequent centrifugation, at "g" between 10000 and 30000, within 90-30 minutes, respectively;
- the supernatant is filtered through a membrane filter with a maximum pore size of 0.45 µm;
- the filtrate is subject to anion exchange chromatography at 2-4°C , with the buffer solution as the moving phase, separating proteins and polypeptides contacting with the adsorbent, with a step-by-step increment, using eluent with ionic strength ranging between 0.08 and 0.26 mol/l and pH - between 5.2 and 8.5, increasing the moving phase ionic strength by increment of 0.02 mol/l, starting to collect target fractions using the solution with ionic strength of at least 0.1; and
- collected target fractions are desalted using the method of dialysis or gel filtration and, after addition of conserving substances characterized by bacteriostatic and fungicide activity with the maximum total concentration of 0.06 mg/ml and solubilizator with the maximum total concentration of 0.01 mg/ml, ultrafiltration with the maximum pore size of 0.22 µm is effected, then sterile packaging is performed.

Another Invention in the claimed group is the biologically active protein polypeptide complex having tissue-specific restoration effect on neural tissues obtained from quick-frozen brain obtainable by the method described in claim 1, including negatively charged, mildly acidic, neutral proteins and polypeptides relating to the factors of growth, differentiation, signaling molecules determining its biological and pharmaceutical activity, with the molecular mass from 5 to 200 kDa, with at least 80% of the total protein mass having molecular mass ranging from 10 to 120 kDa, is characterized with a peak value at the wavelength of 274-284 nm in UV-visible spectrum and presence of bands in the pl range from 4.2 to 8.4 at isoelectric focusing in 5% polyacrylamide gel.

When separating the target product, at the homogenization and anion-exchange chromatography stages, the buffer media pH must be 5.2 to 8.5; the buffer solution must, at the homogenization and extraction stages, have the sufficient concentrations of detergents and proteolysis inhibitors; the anion-exchange chromatography stage must use, as mobile phase, the solutions of the 0.08 to 0.26 mol/l 0.02 mol/l step ionic strength, pH must be 5.2 to 8.5, and the target fractions collection must start with the min. 0.1 ionic strength eluent; the resulting target fractions must contain the 5 kDa to 200 kDa molecular mass proteins and the complex must contain min. 80% of total protein 10 kDa to 120 kDa molecular mass proteins and polypeptides; the resulting complex must be characterized by a peak at the 274-284 nm wave length in the UV-visible spectrum and bands in the pI 4.2 to 8.4 range at isoelectric focusing in a 5% polyacrylamide gel and the proteolytic proteins composing it must have proteolytic activity; and the feedstock must be the quick-frozen mid first third to mid last third gestational age hoof livestock embryonic brain. With other parameters, the complex protein-polypeptide composition and concentration ratios of the forming biologically active components will differ.

The extracted biologically active substance, being a protein-polypeptide complex, differs from the earlier obtained ones extracted from the animal brain, as of the feedstock source at the fixed gestational ages, as of the molecular masses and qualitative composition of the protein-polypeptide fractions, as of the evolutionarily fixed and in ontogenesis growth proteins, differentiation factors, and signaling molecules concentration ratios, and as of the biological and pharmacological activity and this activity specificity. The anion-exchange chromatography extracted protein-polypeptide complex that allows to obtain the eluent ionic strength dependent charge level target fractions consists of the weak-acid and neutral protein and polypeptide components due to the described above empirically combined conditions. A peculiarity of the obtained complex is the existence of the large enough (up to 200 kDa) no allergenicity and no immunotoxicity protein molecules; this is due, first of all, to the feedstock (quick-frozen embryonic brain at certain gestation ages) organo-specific characteristics.

The protein-polypeptide complex feedstock characteristics condition both the availability of the components required for the whole range of biologic activities, i.e., growth factors, tissues and cells differentiation factors, and signaling molecules, and their concentration ratios, as well. Moreover, despite that at different gestational stages the protein-polypeptide composition qualitative composition of the embryonic brain (feedstock) changes, with the claimed method, the target components remaining within the evolutionarily fixed concentration ratios ranges, ensure the required biological activity of the substance. The claimed protein-peptide complex feedstock and production parameters characteristics variations do both deteriorate its biological activity and may lead to adverse events, such as the carcinogenicity, allergenicity, immunotoxicity, etc. The freeze drying of the claimed biologically active substance also significantly (up to 70%) deteriorates its biologic tissue- and organo-specific activity.

### BEST EMBODIMENT OF INVENTION

The following examples illustrate the invention.

### Example 1. Biologically active complex production method.

Protein fraction production from ovine embryonic brain. 200 grams of quick-frozen ovine embryonic brain, 16 to 18 weeks gestational age, is unfrozen and 5-minute homogenized in 1000 ml of 0.05 M TRIS-glycin-phosphate buffer, pH 8.0, containing 1 mM of ethylenediaminotetraacetic acid (EDTA) and 0.1% mannitol, at 4°C. The homogenate is filtered through a close cloth to separate the ballast substances, then 60-minute centrifuged at 20,000 g, then filtered through a 0.45 µm mesh membrane filter at 4°C. The filtrate is then applied to a 200 ml volume chromatographic column with the Toyopearl DEAE-650M anion-exchange medium; the column is equilibrated with 4 volumes of the glycin-phosphate buffer, pH 8.0, containing 0.1 mM of EDTA. The proteins linked to the carrier are separated by the stepwise gradient of the 0.05 M glycin-phosphate buffer, pH 8.0, containing 0.04 M of KCI, 0.02 M-step increasing the salt concentration; the target fractions collection starts at the 0.06 M salt concentration. Chromatography temperature: 2 to 4°C. The target fraction undergoes the stage by stage filtering at max. 8.0 kfg/cm² back pressure, through 5 kDa and 200 kDa retention potential materials; then it is desalinized, and diluted with the 0.05 M glycin-NaOH solution, pH 7.4, down to the 1.2 mg/ml protein concentration, adding the polyoxyethylenesorbite monooleate (Tween 20) to the 0.01 mg/ml total concentration. The solution undergoes the sterilizing filtering, through a max. 0.22 µm mesh membrane filter. To characterize the resulting protein-polypeptide fraction the ultraviolet spectrophotometry, gel chromatography, electrophoresis in polyacrylamide gel, and amino acid analysis were used. The solution ultraviolet spectrum is read in the 250 to 350 nm wave length range, the maximum absorption is observed at 280±5 nm. To determine the molecular mass of the complex forming proteins and polypeptides the following methods are used: gel chromatography with Superdex 75 (GE Healthcare) and denaturing SDS-Page electrophoresis in 12-percent polyacrylamide gel in comparison with a standard marker proteins set. The column is calibrated with the protein standards, molecular masses range: 13 kDa to 250 kDa. The above methods have found that the drug contains the 5 kDa to 200 kDa proteins and polypeptides, of which 82% have the molecular mass within the 10 to 120 kDa range. Amino acid analysis (%): Asp: 10.82 + 2.5; Thr: 5.4 + 1.2; Ser: 5.2 + 1.3; Glu: 16.2 + 3.1; Pro: 7,045 + 2.4; Gly: 5.2 + 2.2; Ala: 5.4 + 1.2; Val: 7.08 + 2.7; Met: 2.65 + 1.3; He: 4.45 + 1.5; Leu: 9.4 + 2.2; Tyr: 4.02 + 1.1; Phe: 4.8 + 1.3; Orn: 0.48 + 0.1; Lys: 8.48 + 2.1; His: 2.8 + 0.8; Arg: 6.52 + 2.1.

### Example 2. Biologically active complex production method.

Protein fraction production from the porcine embryonic brain. 200 grams of quick-frozen porcine embryonic brain, 3 to 4 weeks gestational age, is unfrozen and 5-minute homogenized in 800 ml of 50 mM TRIS-glycin-phosphate buffer, pH 5.8, containing 1 mM of ethylenediaminotetraacetic acid (EDTA) and 0.1% maltose, at 4°C. The homogenate is filtered through a close cloth to separate the ballast substances, then it is 30-minute centrifuged at 30,000 g, and then filtered through a 0.45 µm mesh membrane filter at 25°C. The filtrate is then applied to a 250 ml volume chromatographic column with the DEAE-Sepharose anion-exchange medium; the column is equilibrated with 4 volumes of the maleate buffer solution, pH 5.8, containing 0.1 mM of EDTA. The proteins linked to the carrier are separated by the stepwise gradient of the maleate buffer solution, pH 8.0, containing 0.04 M of NaCl, 0.02 M-step increasing the salt concentration; the target fractions collection starts at the 0.06 M salt concentration. Chromatography temperature: 2 to 4°C. The target fraction is stage by stage ultra filtered at max. 8.0 kfg/cm² back pressure, through 5 kDa and 250 kDa retention potential; then desalinized, and diluted with the 50 M aspartate-NaOH solution, pH 7.4, down to the 1.2 mg/ml protein concentration, adding the polyoxyethylenesorbite monooleate (Tween 20) to the 0.005 mg/ml total concentration. The solution is sterilizing filtered, through a max. 0.22 µm mesh membrane filter. To characterize the resulting protein-polypeptide fraction the ultraviolet spectrophotometry, gel chromatography, electrophoresis in polyacrylamide gel, and amino acid analysis were used. The solution ultraviolet spectrum is read in the 250 to 350 nm wave length range, the maximum absorption is observed at 280±5 nm. To determine the molecular mass of the complex forming proteins and polypeptides the following methods are used: gel chromatography with Superdex 75 (GE Healthcare) and denaturing SDS-Page electrophoresis in 12-percent polyacrylamide gel in comparison with a standard marker proteins set. The column is calibrated with the protein standards, molecular masses range: 13 kDa to 250 kDa. The above methods have found that the drug contains the 5 kDa to 200 kDa proteins and polypeptides, of which 82% have the molecular mass within the 10 to 120 kDa range. Resulting complex amino acid analysis (%): Asp: 10.82 + 1.3; Thr: 5.4 + 0.9; Ser: 5.2 + 1.1; Glu: 16.2 + 1.9; Pro: 7.045 + 1.7; Gly: 5.2 + 0.8; Ala: 5.4 + 1.1; Val: 7.08 + 2.3; Met: 2.65 + 0.6; He: 4.45 + 0.8; Leu: 9.4 + 2.5; Tyr: 4.02 + 0.6; Phe: 4.8 + 1.1; Orn: 0.48 + 0.1; Lys: 8.48 + 2.3; His: 2.8 + 0.7; Arg: 6.52 + 2.1.

### Example 3. Effect of biologically active complex (drug) on brain explants development

The experiments used 52 fragments of the brain cortex and 40 fragments of the cerebrospinal ganglions of the 10- to 12-day gallinacean embryos. The nutrient medium to cultivate the explants contained: Eagle's solution: 35%, bovine fetal serum: 25%, Hanks' solution: 35%, and gallinacean embryonic extract: 5%; glucose (0.6%), insulin (0.5 U/ml), penicillin (100 U/ml), and glutamine (2 mM) were added. The brain cortex and cerebrospinal ganglions fragments were placed in this medium and cultivated 2 days on collagenic substrate, in Petri dishes, in thermostat, at 36.7°C. The protein-polypeptide complex under study and the 0.5, 1, 2, 10, 20, 50, 100, 200, 400, 800, and 1000 ng/ml Cerebrolysin and Cortexin as positive control were added to the experimental medium. The area index, i.e., the total explant with the growth zone area to the brain fragment outgoing area ratio served as biological activity criterion. The difference reliability of the compared mean area indexes was assessed using the Student's t-criterion. The area index was expressed in percents; the control area index was assumed 100%. The growth zone of the control brain explants was formed by the short neurites, glia progenitor cells, and fibroblast-like cells. The following experimental series were set up when studying the direct effect of the drug on the brain fragments. Cerebrolysin and Cortexin in various concentrations were added to the gallinacean embryos brain cortex and cerebrospinal ganglia explants nutrient medium. On the 3rd day of cultivation with the 100 ng/ml Cerebrolysin addition the reliable brain cortex explants area index augmentation by 24±3.5% was observed, compared with the control area indexes. No reliable brain cortex explants area indexes were observed at other Cerebrolysin concentrations. On the 3rd day of cultivation with the 50 ng/ml Cortexin addition the reliable brain cortex explants area index augmentation by 32±4% was observed, compared with the control area indexes. A reliable brain cortex explants area index augmentation was observed at: Cortexin 100 ng/ml: by 28+3.5%, and Cortexin 200 ng/ml: by 23+2%. No reliable brain cortex explants area indexes were observed at other Cortexin concentrations. A distinct brain cortex explants development activation under the 20 ng/ml complex under study was observed: the experimental explants area index was by 48±4.5% greater than that of the control explants and comparison explants under the Cortexin and Cerebrolysin. No reliable explants area index augmentation was observed when adding the Cerebrolysin into the cerebrospinal ganglions explants cultivation medium; with the Cortexin, the explants area index augmentation by 22±3% was observed. With the complex under study addition into the cerebrospinal ganglions explants cultivation medium the explants area index augmentation by 36±3.5% was observed. When studying the longer, 7-day, brain cortex and cerebrospinal ganglions explants cultivation, the same neurite-stimulating effects were observed with the same complex under study concentrations. Thus, as of the brain tissues, the lower efficient protein-polypeptide complex under study concentrations threshold was observed compared with the Cerebrolysin and Cortexin. Thus, the brain cortex explants fragments growth zone augmentation was observed only for the 100 ng/ml Cerebrolysin and 50 and 100 ng/ml Cortexin; for the protein-polypeptide complex under study the respective concentrations were 20, 10, 50, and 100 ng/ml. That said, the protein-polypeptide complex stimulating effect intensity was reliably higher than for the Cortexin and Cerebrolysin. No reliable Cerebrolysin effect on the cerebrospinal ganglions explants growth zone augmentation was observed; the Cortexin reliably increased the area index by 24%, while the protein-polypeptide complex under study provoked the area index augmentation by 36%. This shows the well expressed and directed stimulating effect of the drug on different brain divisions neurons.

### Example 4. Biologically active complex toxicity study.

The protein-polypeptide complex systemic toxic effects were studied in the acute toxicity conditions, single administration, and chronic toxicity, long-term administration [5]. The acute toxicity study used 72 outbred male white mice, 20 to 22 grams body mass. The animals were at random split into 6 equal groups. The animals received one fold intramuscular injections of 5 mg/kg, 10 mg/kg, 100 mg/kg, 200 mg/kg, and 500 mg/kg complex in 0.5 ml of sterile 0.05 M glycin-NaOH pH 7.5 solution. The control group animals received the same doses of the sterile 0.05 M glycin-NaOH pH 7.5 solution. The subacute toxicity study used 95 outbred male white rats, 150 to 180 grams body mass. The animals received the protein-polypeptide complex once daily intramuscularly, 1 mg/kg, 10 mg/kg, 50 mg/kg, and 100 mg/kg in 0.5 ml of sterile 0.05 M glycin-NaOH pH 7.5 solution. The control group animals received the same doses of the sterile 0.05 M glycin-NaOH pH 7.5 solution. The peripheral blood morphologic composition and properties were studied before the complex introduction and on the 30th, 60th, and 90th days. Upon the experiment completion, the blood biochemistry and coagulology were measured.

The chronic toxicity study used 112 outbred male guinea pigs, 250 to 680 grams body mass, during 6 months. The experimental groups animals received the protein-polypeptide complex once daily intramuscularly, 1 mg/kg, 10 mg/kg, 50 mg/kg, and 100 mg/kg in 0.5 ml of sterile 0.05 M glycin-NaOH pH 7.5 solution, during 6 months. The control group animals received the same doses of the sterile 0.05 M glycin-NaOH pH 7.5 solution. The erythrocytes, hemoglobin, reticulocytes, thrombocytes, and leucocytes numbers, leucocyte formula, erythrocyte sedimentation rate (ESR), and erythrocyte resistance were determined in the animals' peripheral blood using conventional methods. In addition, the total protein in blood serum (Lowry's method), and potassium and sodium (plasma spectophotometry) were determined. Upon completion of the experiment the pathomorphological exams of the brain and spinal marrow, spinal ganglions, thyroid and parathyroid glands, adrenal glands, seminal glands, hypophysis, heart, lungs, aorta, liver, kidneys, urinary bladder, pancreatic gland, stomach, small bowel, large bowel, thymus, spleen, lymphatic glands, and bone marrow were performed. The acute toxicity studies have revealed that the single administration of the complex under study in animals, in the dose exceeding the therapeutic one recommended for clinical use more than 5000-fold does not provoke toxic reactions; this means a large therapeutic range of the complex. The complex subacute and chronic toxicities studies have revealed no side effects at the long term administration in the doses exceeding the supposed therapeutic one 3000-fold. The exam of the complex effect on the guinea pigs peripheral blood morphology and biochemistry 3 and 6 months after the study start has revealed no reliable alteration of these parameters.

No pathological changes have been revealed when assessing the animals' performance status, peripheral blood morphology and biochemistry, internals, cardio-vascular and respiratory system, and liver and kidneys functions. Hence, the protein-polypeptide complex obtained by the claimed method does not have, at long term administration to animals, any toxic properties preventing from its use as a parenteral drug.

### Example 5. Substance mutagenicity.

To assess the claimed protein-polypeptide complex mutagenicity, the following set of tests was used:
- Ames Salmonella/microsome mutagenicity assay using the TA 97, TA 98, and TA 100 *Salmonella typhimurium* strains as test objects; and
- micronuclei in murine bone marrow cells.

A protein-polypeptide complex sample was tested, in form of transparent liquid sterile packed in a dark glass vial, 20 ml, 2.3 mg/ml substance.

### AMES TEST PROTEIN-POLYPEPTIDE COMPLEX MUTAGENICITY ASSESSMENT.

The *Salmonella typhimurium* mutagenicity assay is a bacterial test system to account for the histidine prototrophy gene mutations under effect of the chemical compounds and/or their metabolites, inducing the mutations of the base substitution or reading frame shift in organism genome type. The mutagens inducing the base pairs substitution are the agents provoking the base pairs substitution mutations in the DNA molecule. The mutagens inducing the genetic code reading frame shift mutations are the agents provoking the addition or deficit of single or multiple base pairs in the DNA molecule.

The method's purpose is to reveal the capability of the pharmaceutical substances or their metabolites to induce the gene mutations in the *Salmonella typhimurium* indicator strains. The bacteria are treated with a compound under test with (SM+) or without (SM-) metabolic activation system. After a certain time incubation, the different test strains revertant colonies number is compared with that of the negative control spontaneous revertant (untreated or solvent treated cultures). If the compound under test and/or its metabolites have mutagenic activity, they will induce the reverse mutations from histidine auxotrophy to prototrophy in the *Salmonella typhimurium* histidine-dependent strains.

The tests used a set of the *Salmonella typhimurium* indicator strains allowing to register the genetic code reading frame shift (TA 98 and TA 97) base pair substitution (TA 100) mutations. The strains originate from the Russian National Collection of Industrial Microorganisms, Research Institute for Genetics and Selection of Industrial Microorganisms, Scientific Center of Russian Federation.

The bacterial cultures handling Rules of procedure, the strains genotype checks, their museum keeping rules, the experimental utensils, reagents, nutrient media and solutions, rat liver homogenate getting, activating mix compounding, and statistical analysis methods complied with the standards described in detail in the relevant literature.

The metabolic activation used the S9 fraction of the Wistar male rats liver; 5 days before sacrifice the rats received the microsomal enzymes inductor (sovol, 300 mg/kg, single, intraperitoneally). To control the metabolic activation system the ethidium bromide (10 µg/dish), TA 98 strain, SM+, was used.

The 2.3 mg/ml protein sterile protein-polypeptide complex solution was examined: 0.3 and 0.1 ml/dish mother solution and three 10X dilution in physiological solution (0.1 ml/dish). The substance test doses were 690, 230, 23, 2.3, and 0.23 µg/dish.

The experiment had positive controls; for them the mutation-inducing substances in relevant microorganisms strains with or without activation were used. The no-activation used the sodium azide, 10 µg/dish, TA 100 strain, SM-; 2,7-2,7-diamino-4,9 dioxy-5,10-dioxo-4,5,9,10-tetrahydro-4,9- diazopyrene (DDDTDP), 10 µg/dish, TA 98 strain, SM-; and 9-aminocaridine (9AA), 50 µg/dish, TA 97 strain, SM-. To control the metabolic activation system activity the ethidium bromide (10 µg/dish), TA 98 strain, SM+, was used. The negative control used the physiological solution (0.1 ml/dish).

The selective semi-enriched agar (0.7%) in test tubes was melted in water bath at 100°C; then it was placed into the 45°C to 46°C thermostated water bath. First, 0.1 ml of the relevantly diluted sample, then 0.1 ml of the bacterial suspension and 0.5 ml of the microsomal activating mix (metabolic activation variant) were put into the agar test tubes. Then the tube content was quickly blended and discharged onto the lower minimal agar in the Petri dishes. The microsomal activating mix and semi-liquid agar reserve introduction time per dish did not exceed 10 to 15 seconds. The dishes were left for 30 to 40 minutes at ambient temperature; after the first agar gelation they were put into a 37°C thermostat. The results were accounted after a 48-hour incubation.

The no metabolic activation system (SM-) and metabolic activation system (SM+) variants were experimented in parallel. In the SM- variant the direct mutagens (drugs mutagenic due to the compound initial structure activity) effect may be observed. As of the effect of the promutagens, i.e., the compounds whose effect associates with the mutagenic metabolites formation, this may be accounted when comparing the results of the SM- and SM+ compounds tests analysis data.

Every control and experimental variants used 2 dishes each. The mutagenic effect was considered significative when the mean number of the revertants colonies per dish in experiment exceeded the control one 2- and more fold. The experimental results were taken into account at the condition of standard response in all the positive and negative control variants. The revertants colonies numbers in the solvent control, SM- and SM+ variants, were within the spontaneous level fluctuations range for the given strains. The strains response to the standard mutagens was within the common levels range.

The examined with all concentrations protein-polypeptide complex has not shown any mutagenic effect for the TA 100, TA 98, and TA 97 strains, with and without metabolic activation system.

CONCLUSION: the protein-polypeptide complex is unable to induce gene mutations with the *Salmonella typhimurium* test strains within all the experimental concentrations range.

### MICRONUCLEAR TEST PROTEIN-POLYPEPTIDE COMPLEX MUTAGENICITY ASSESSMENT IN MAMMALIA CELLS.

The cytogenetic activity is the capacity of a substance to provoke structural and quantitative chromosomal abnormalities in the somatic and embryonic cells.

The micronuclei are the small size DNA-containing formations consisting of the chromosomal acentric fragments or lagged in ana-telophase chromosomes. At the telophase stage these fragments may get into the daughter cells nuclei or form individual or multiple micronuclei in cytoplasm.

The Study purpose is to reveal and quantitatively assess the cytogenetic activity (mutagenicity) of the pharmacologic drugs in the mammalian bone marrow polychromatophils. The method's base is the micronuclei cells microscopic registration.

The experimental used the male and female F1(CBAx C57BI6/J) hybrid mice, two-month age, 18 to 20 grams body weight. Every group included 6 animals. The animals were kept under the 12-hour light regime, with freely available water and food. The single and five fold administration protein-polypeptide complex mutagenicity assessment animal experiments were in compliance with the official protocols. Three experimental series with relevant controls were performed: therapeutic dosage: males, single administration; subtoxic dose: males, single administration; and therapeutic dosage: males and females, five fold administration.

Taking into account that the possible biologically active protein-polypeptide complex clinical administration method may be intravenous or endolumbar, this Study used the intraperitoneal one with mice. The protein-polypeptide complex mutagenicity assessment dose was defined on the basis of the maximum recommended daily therapeutic dose (TD) for human. The 2 ml at 0.5 mg/ml concentration IV administration is recommended. The therapeutic dose for human, including infant, may be 0.05 mg/kg/day. To calculate the mice experimental dose, the conversion factor is recommended to take into account the human and murine body surface area to body mass ratio. In our study it was 8.33. Hence, for the murine therapeutic dose the approximately 8.33 human therapeutic dose was taken (0.42 mg/kg).

For the subtoxic dose the maximum possible experimental one was used, 23 mg/kg, as the substance concentration was 2.3 mg/ml; the conventional total substance administered to mice is 0.1 ml per 10 g body weight. hence, calculated for human, the maximum tested dose was 2.76 mg/kg.

Seven groups of animals, four experimental and three control ones, were formed (see Table 2). The 0.42 mg/kg substance was five times, at 24-hour intervals, administered to both the males and females. In all the groups the animals were sacrificed 6 hours after the last protein-polypeptide complex administration.

In two other experimental groups, the protein-polypeptide complex was administered once to the males, at 0.42 mg/kg and 23 mg/kg.

For the negative control the negative control the physiological solution was used. It was administered intraperitoneally to the males and females of the two control groups, the times and volumes were the same as for the experimental. Positive control: 20 mg/kg cyclophosphamide dissolved ex tempore in physiological solution, single intraperitoneal administration, 24 hours before sacrifice.

The bone marrow cells preparation for the micronuclei account were made in conventional manner, in accordance with the "Micronuclear Test Environmental Factors Mutagenicity Assessment in Mammalia Organs Cells" Guidelines. The sacrifice method was the cervical dislocation 6 hours after last drug administration. Both femoral bones were extracted, muscles were then removed. To wash out the bone marrow the bovine embryonic serum (NPP PanECO). The bone marrow from the both femoral bones was extracted into a microtube with a syringe. The suspension was 5-minute centrifuged at 1000 rpm, the supernatant was removed, and the sediment was carefully resuspended. A drop of the suspension was used to prepare the smear then dried in air. The smear was then methanol fixed during 3 minutes. For staining the Romanowsky-Giemsa method was used. 2000 polychromatophils per animal were then analyzed with coded preparations. At the 500 erythrocytes count the polychromatophil to normochromatic erythrocytes ratio was determined. Analysis finished, the preparations were coded.

The positive result criterion is the reproducible and statistically significant growth of the micronuclei polychromatophil erythrocytes number in at least one experimental group compared with the control one. The positive result indicates that the substance induces the chromosomal damages and/or cells mitotic apparatus damages in experimental animals.

The micronuclei account results statistical treatment used the X2 criterion experimental to control series comparison; the polychromatophils proportion intergroup comparison used the Mann-Whitney criterion.

Mice bone marrow micronuclei polychromatophils account results: in no experimental variants the protein-polypeptide complex induced the mice bone marrow micronuclei polychromatophils proportion augmentation as compared between the experimental and relevant control series. The 20 mg/kg cyclophosphamide (positive control) intraperitoneally in male mice provoked 19.6 fold higher micronuclei cells (62.1‰ vs. 3.16‰ in control, P < 0.001). The polychromatophil erythrocytes share in the total bone marrow erythrocytes was at approximately the same level for both the experimental and control groups; hence, no toxic effect of the substance in tested doses on hematogenesis. Under the positive control (cyclophosphamide) the polychromatophil to normochromatic erythrocytes ratio shift to the latter ones was recorded (compared with control, P < 0.005).

Thus, the test for the micronuclei induction in the mice bone marrow micronuclei polychromatophils at one and five fold 0.42 mg/kg body weight intraperitoneal administration to males and females (corresponding, as recounted, to the daily therapeutic dose for human) has not revealed any protein-polypeptide complex mutagenic activity. No mutagenic activity has been revealed for the maximum possible, 23 mg/kg, single protein-polypeptide complex administration in males. The protein-polypeptide complex has not shown any toxic effect as of the "polychromatophil erythrocytes proportion" parameter.

### CONCLUSION FOR THE PROTEIN-POLYPEPTIDE COMPLEX MUTAGENICITY ASSESSMENT

The protein-polypeptide complex has shown no mutagenic activity in either the Ames Salmonella/microsomes test or the mice bone marrow cells micronuclei induction test performed in accordance with the Guidelines for Experimental (Preclinical) Study of New Pharmacological Substances.

### Example 6. Substance specific activity [6, 7].

### LOCAL IRRITANT EFFECT.

The studies have shown that the biologically active protein-polypeptide complex substance gives no local irritant effect when administered subcutaneously, intravenously, and intranasally. At the rapid intravenous administration the hyperthermia effects are possible. No local irritant effect on the nose and throat mucosa has been revealed at the 1-month intranasal administration in rabbits.

### IMMUNOTOXICITY AND ALLERGENICITY STUDIES

### Active cutaneous anaphylaxis

The experimental used the guinea pigs, 10 per group, 3 groups. Sensibilization pattern: first injection subcutaneously; then two intramuscularly, alternate days, femoral area. On the 14th day, the substance was administered intracutaneously, in two fold dilution, on sheared areas of the back. To control the cutaneous sensibility, the physiological solution was administered to a same animal, on another sheared skin area. The control animals were administered an injection boosting substance (equal to the sensibilizing dose). Then the animals were intravenously administered 0.5 ml 1% Evans blue solution. Thirty minutes later the animals were sacrificed with ester; the blue spot on inner skin side at the administration point size was determined. The reaction was considered positive at the larger than 6 mm spot vs. max. 3 mm in control. In 3 cases the positive reaction was recorded in the group having received a 10-fold therapeutic dose. The number of reactions means the possibility of individual responses.

### Delayed type hypersensitivity reaction in mice.

The experimental used the nonlinear, 18 to 20 g body mass mice, 10 animals per group, 30 in total. The sensibilization was single, intracutaneously, at tailset, drug emulsion in NAF (1:1). The dose administered was 60 µl. The control animals were sensibilized with the PAF emulsion with Hanks' solution (1:1). To reveal the sensibilization, 5 days after, the mice were administered 40 µl of the test drug in Hanks' solution into a hind foot pad. Twenty four hours after test, the edema size was measured using the MK-0-25 engineering micrometer. The results statistical treatment has not revealed any significant difference in the feet thickness between the experimental and control groups (P > 0.05). The substance administration does not provoke the delayed type hypersensitivity reaction.

### Peritoneal macrophages phagocytic activity study.

The experimental used the 180 to 200 g body weight albino rats, 30 animals, 10 per group. The two drug doses, therapeutic and 10-fold therapeutic ones, were studied; the third group was for control. The method is based on determining the lysing solution optical density after destruction of the phagocytes having absorbed the neutral red particles. The rats exposed to the drug under study were decapitated. The animals were intraperitoneally administered 5 ml of the 199 medium containing 20% of the bovine embryonic serum. Having massaged the peritoneal cavity, the medium was sucked out with a syringe. The portions of the cellular suspension obtained from the whole group of animals were joined in a common pool. The living cells concentration was brought to 1.5 x 10³ cells/ml. The cellular suspension was then sterilely dispensed into the 40 mm diameter plastic Petri dishes, 2 ml each. The dishes were 2-hour incubated at 37°C. The supernatant with unstuck cells was disposed; the monolayer fixed to the plastic was twice washed with the 199 medium. A solution with the neutral red was put into the slightly dried dishes; these were then cured 1 hour at 37°C. The solution was poured off. The cells were washed with the 199 medium. Three milliliters of the lysing solution was put into every dish. The cells were thoroughly treated by rotating motion. The solution was poured into test tubes; the optical density was checked with spectrophotometer at 540 nm wave length.

The substance administration does not inhibit the macrophages activity. No statistically valid differences exist between the experimental and control groups results.

### Mast cells degranulation reaction

The experimental used 30 female rats 180 to 200 g body weight. The substance solution was administered intraperitoneally in the therapeutic and 10-fold therapeutic doses; the intact group served as control. To obtain the mast cells suspension, the animals were decapitated, a buffer was injected into the peritoneal cavity; having massaged the anterior abdominal wall, the mast cells suspension was collected in centrifugal test tubes. The preparations were made on the glass slides stained with the 0.3% neutral red in alcohol solution. To 0.03 ml of the mast cells suspension, 0.03 ml of the experimental animal serum and 0.03 ml of the drug under study in 1:100 dilution were added. In control the maximum degranulation was 5%. The preparations were covered with cover glass sealing the edges with wax. Then they were 15-minute incubated in thermostat at 37°C. The preparations were studied under microscope at X40, counting the degranulated and normal mast cells, 100 in total. The reaction was considered positive with the degranulation above 20%.

The substance in the 0.01 mg/kg concentration does not provoke the mast cells degranulation, is the same for the control; the 5% threshold has not been overpassed. The ten fold therapeutic dose group reaction is feebly positive, however, within the statistical errors percentage.

### PRIONS TESTS.

The assays were taken from 4 vials. The reaction used the enzyme-linked immunosorbent assay method, with a 96-socket polystyrene pad. Diagnostic test system: PrionScreen. Spectrophotometer: Digiscan. Every assay underwent 4 repeats. Controls: K-: 8 sockets, K+: 8 sockets. The resulting control and study optical densities were consistent with the reaction conditions.

Results: no prions revealed in substance under study.

### GENERAL CONCLUSION.

The biologically active protein-polypeptide complex original substance toxicity in the acute and subchronic experiments, and its local irritant, immunotoxic, and allergenic effects have been studied; and search for possible prion infections has been conducted.

The drug acute toxicity has been experimentally studied with the BALB/C line mice. The drug was administered subcutaneously, intraperitoneally, and per os. The maximum possible for administration in mice dose is 0.2 ml, making 10 mg/kg. It has been revealed that in the specified dose the drug administered by any of the three methods did not result in the animals' death or provoke acute poisoning.

The subchronic experiment used the Wistar line rats, both mature and immature. The drug under study was administered subcutaneously, 0.01 mg/kg therapeutic and 0,1 mg/kg ten fold therapeutic doses; and intravenously, 0.0025 mg/kg, and x10 0.025 mg/kg. The intranasal experiment, 1 shot/day (or 1.5 mg/ml, 0.2 ml) used the Chinchilla rabbits. The toxicity study used the conventional methods for the blood hematology and biochemistry, and viscera histology.

It has been revealed that the substance administration by any way, in both the therapeutic and ten-fold therapeutic doses, in the course of 1 month, provokes no viscera alteration and gives no toxic effect on the blood system. No local irritant effects have been revealed at any administration.

The allergenicity studies used the intracutaneous applications in guinea pigs. The substance has not provoke any allergic response in therapeutic doses. For the ten fold therapeutic dose, three cases of hypersensitivity have been recorded. No immunotoxicity with the Wistar rats has been revealed.

The prion infections search used the enzyme-linked immunosorbent assay method. The drug is free of these causative agents.

### Example 7. Effect on cell population cytokinetic parameters.

**Materials and Methods.** The 3T3 murine fibroblasts were cultivated on the DMEM (PanEco, Moscow, Russia), with additions: 584 mg/l glutamine; antifungal-antimicrobial agent (Sigma, USA) according to manufacturer's instruction, and 10% vol. standardized bovine embryonic serum (HyClone, USA). For experimental, the cells were planted onto the 24-socket pads (Corning), 3000 cells/ml (socket). For morphology the cells were planted onto the 12 mm diameter cover glasses (Ted Pella, USA), placed in the sockets.

**Test substances application.** A cell culture having reached the required density, the culture medium was replaced with the fresh one of the same composition (control), DMEM without serum with 2 mg/ml of BSA (Sigma) medium (negative control), DMEM without serum with 10% vol. biologically active substance (experiment 1), and DMEM with 10% serum and 10% biologically active substance (experiment 2.

**Experimental procedures** The cells were analyzed 8, 24, and 48 hours after the test agents application. The culture in vivo state was analyzed; the cells photos were taken: Axiovert 200M phase-contrast inverted microscope (Carl Zeiss, Germany), with EC Plan-Nefluar 10x NA 0.5 field lens, and ORCA II Erg-2 digital camera (Hamamatsu Photonics, Japan). To determine the cells number, the cells sheet was dissociated with the Versen solution (PanEco) and 0.025% trypsin solution (PanEco) 3:1 mix. The resulting suspension was centrifuged at 300 g; the sediment was resuspended in the Hanks' solution, and counted in the Goryaev chamber. To analyze the cell population cytokinetic parameters, the cells cultivated on the cover glasses were fixed with the 1% gluteraldehyde on 0.1 M phosphate buffer at 4°C; permeabilized with the 0.5% Triton X-100 solution on PSB (10 minutes); washed with the distilled water; stained with the Mayer's hematoxylin (90 seconds) (Sigma); desiccated; and put into DePeX. After permeabilization, the cells were stained with the DAPI (0,1 µg/ 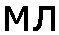 ) and put into Moviol. The resulting preparations were studied under the Axioskop II (Carl Zeiss) fluorescence microscope, Plan-Neofluar 40x NA 1,2 and 100x NA 1,3 field lenses.

### RESULTS. POPULATION CYTOKINETIC PARAMETERS.

*Control.* 8 hours. Mitotic index: 8%. Apoptosis and necrosis: 0%.
24 hours. Mitotic index: 11.9%. Apoptosis and necrosis: 0%. Cells in socket: 265,000.
48 hours. Mitotic index: 9%. Apoptosis and necrosis: 0%. Cells in socket: 780,000.
*BSA.* 8 hours. Mitotic index: 5%. Apoptosis and necrosis: 0%.
24 hours. Mitotic index: 5.5%. Apoptosis and necrosis: 0%. Cells in socket: 167,500.
48 hours. Mitotic index: 3%. Apoptosis and necrosis: 0%. Cells in socket: 625,000.
   *Biologically active substance.*
8 hours. Mitotic index: 6%. Apoptosis and necrosis: 0%.
24 hours. Mitotic index: 7.1%. Apoptosis and necrosis: 0%. Cells in socket: 250,000.
48 hours. Mitotic index: 6%. Apoptosis and necrosis: 0%. Cells in socket: as counted: 187,000; actual: ≈ 400,000 (ejection).
   *Serum* + *Biologically active substance.*
8 hours. Mitotic index: 8%. Apoptosis and necrosis: 0%.
24 hours. Mitotic index: 4.5% (ejection). Apoptosis and necrosis: 0%. Cells in socket: 317,500.
48 hours. Mitotic index: 9%. Apoptosis and necrosis: 1%. Cells in socket: 680,000.

All the experimental lines show the biomass augmentation; no cytotoxic effect is observed in any case. In the negative control, the proliferation efficiency is minimal; with the protein-polypeptide complex the proliferation is stimulated compared with the negative control. The positive control and substance and serum added medium show a same proliferation level.

The cells incubated with the substance form thin and long processes. This is especially well seen in the first experimental day. With the serum on the substance background the effect remains.

### Conclusions:

1) The biologically active substance consisting of the protein-polypeptide complex is not a cytotoxic agent.
2) The substance contains growth factors, but is not as efficient as proliferation stimulator as the blood serum.
3) The substance provokes the cells shape changes, with a well visible processes formation.

### Example 8. Effect on brain blood supply and rats survival rate under brain ischemic damage.

The experimental used the Wistar male rats, 180 to 250 g body weight, under irreversible bilateral occlusion of the common carotid arteries, using the generally known methods [8-10]. All the veterinary manipulations were aseptic, to keep the experimental animals status. The experimental animals were kept in standard conditions [11].

The rats received the ether (chemically pure diethyl ether, OAO "Medhimprom") anesthesia; then a 8 to 10 cm long skin discission was made in the neck region, along the median line, to access the carotid arteries. The both carotid arteries were exposed, separating the vagus and sympathetic nerves. The silk threads (Silk blk 17 x 45 cm/M3/USP2/ Non needled/ Ethicon Johnson & Johnson, cat. No W203) were pulled under the vessels; then, together with an assistant, the both carotid arteries were single-step ligated with triple friction knot. The skin discission was then sutured with the surgical silk (Silk blk 100 cm/M5/Sgle armed KP-3USP2/ Non needled/ Ethicon Johnson & Johnson, cat. No W794), one or two surgical stitches, with further wound treatment with the 5% iodine tincture. The physiological solution or cellex were administered intraperitoneally. The total manipulations time was 8 to 10 minutes; then the animals were placed into cage. The survival rate was followed up immediately after the procedure and in the course of the first day, i.e., the final survival rate was fixed in 24 hours. The drug biological activity in form of the brain protection under incomplete global ischemia at single-step carotid arteries ligation was assessed as follows:
- **expressed:** survival rate: > 80% rats;
- **median expressed:** survival rate: > 70% to 80% rats; and
- **unexpressed:** survival rate: < 70% rats.

The data statistical processing used the Fischer exact test, accounting for the comparisons multiplicity.

### Experiment 1.

Three groups of animals were used:
- **Group 1:** control animals, physiological solution with sterile disposable insulin syringe: 0.5 ml/rat in 1 hour and 0.5 ml/rat in 5 hours after carotid arteries ligation;
- **Group 2:** sham operated animals: same manipulations as in control but without carotid arteries ligation; physiological solution 0.5 ml/rat in 1 hour and 0.5 ml/rat in 5 hours; and
- **Group 3:** animals after carotid arteries ligation; biologically active substance: 0.5 ml/rat 0.1% solution (0.2 mg/kg) in 1 hour and 0.5 ml/rat in 5 hours.

The experiments have shown that the greatest number, i.e., 11 of 24, of the animals in control died within the 1st hour after the carotid arteries ligation; 4 more animals died within the next 24 hours; in total, 15 rats of 24 died (63%, see Table 1). Among the sham operated animals 2 (17%) rats died immediately after the operation. The biologically active substance 0.2 mg/kg (0.5 ml 0.1% solution) intraperitoneally in 1 and 5 hours provoked death in only two animals: 34/36 rats survived. The compared to control survival rate augmentation is 95%. Table 1 shows the obtained data.

**Table 1. Effect of biologically active substance on rats survival rate under ligation of both carotid arteries**

| Groups of animals | Expressed bioactivity | |
|---|---|---|
| | survived | died |
| Group 1 Control: 24 rats | 9 of 24 (37%) | 15 of 24 63% |
| Group 2 Sham operated 12 rats | 10 of 12* (83%) | 2 of 12 (17)% |
| Group 3 Substance: 0.2 mg/kg 36 rats | 34 of 36* (95%) | 2 of 36 (5%) |

| | | |
|---|---|---|
| * -P ≤ 0.01 compared with control. | | |

Hence, the protein-polypeptide complex biologically active substance ensures a statistically significant animals survival rate augmentation compared with control under incomplete global ischemia of brain at single-step both carotid arteries ligation.

### Experiment 2.

Three groups of animals were used:
- **Group 1:** control animals, physiological solution with sterile disposable insulin syringe: 0.5 ml/rat in 1 hour and 0.5 ml/rat in 5 hours after carotid arteries ligation;
- **Group 2:** animals after carotid arteries ligation; biologically active substance: 0.5 ml/rat 0.1% solution (0.2 mg/kg) in 1 hour and 0.5 ml/rat in 5 hours; and
- **Group 3:** animals after carotid arteries ligation; 0,2 mg/kg freeze-dried biologically active substance: 0.1 mg dry weight in 0.1% physiological solution, 0.5 ml/rat in 1 hour and 0.5 ml/rat in 5 hours.

The experiments have shown that the greatest number, i.e., 11 of 24, of the animals in control died within the 1st hour after the carotid arteries ligation; 4 more animals died within the next 24 hours; in total, 15 rats of 24 died (63%, see Table 1). The diluted freeze-dried biologically active substance 0.2 mg/kg (0.5 ml 0.1% solution) intraperitoneally in 1 and 5 hours provoked death in only four animals: 8/12 rats survived. The biologically active substance 0.2 mg/kg (0.5 ml 0.1% solution) intraperitoneally in 1 and 5 hours provoked death in only two animals: 34/36 rats survived. The compared to control survival rate augmentation is 95%. Table 1 shows the obtained data.

**Table 1. Effect of biologically active substance on rats survival rate under ligation of both carotid arteries**

| Groups of animals | Expressed bioactivity | |
|---|---|---|
| | Survived | Died |
| Group 1 Control: 24 rats | 9 of 24 (37%) | 15 of 24 63% |
| Group 2 Dried cake 12 rats | 8 of 12* (66,7%) | 4 of 12 (33,3)% |
| Group 3 Substance: 0.2 mg/kg 36 rats | 34 of 36* (95%) | 2 of 36 (5%) |

| | | |
|---|---|---|
| * -P ≤ 0.01 compared with control. | | |

Hence, the protein-polypeptide complex biologically active substance ensures a statistically significant animals survival rate augmentation compared with control under incomplete global ischemia of brain at single-step both carotid arteries ligation.

The protein-polypeptide complex freeze-dried fraction biological activity under incomplete global ischemia of brain at single-step both carotid arteries ligation is much lower than that of the liquid substance.

Taken into account sources of information:
1. Encyclopedia of Drugs, 2006, p. 970.
2. RF Patent No 2128511(1999)
3. RF Patent No 2104702(1999)
4. RF Patent No 2049472(1999)
5. Methodological Guidelines. Carcinogenicity Assessment of Pharmacological Drugs and Excipients in Short-Term Tests. // Guidelines for Experimental (Preclinical) Study of New Pharmacological Substances. General editor: Prof. R.U. Khabriev, RAMS. 2nd ed., rev. and corr. Moscow, OAO "Meditsina" Publishing House. 2005., p.131-170.
6. Methodological Guidelines. Carcinogenicity Assessment of Pharmacological Drugs. // Guidelines for Experimental (Preclinical) Study of New Pharmacological Substances. General editor: Prof. R.U. Khabriev, RAMS. 2nd ed., rev. and corr. Moscow, OAO "Meditsina" Publishing House. 2005., p.100-122.
7. Methodological Guidelines. Carcinogenicity Assessment of Pharmacological Drugs and Excipients in Short-Term Tests. // Guidelines for Experimental (Preclinical) Study of New Pharmacological Substances. General editor: Prof. R.U. Khabriev, RAMS. 2nd ed., rev. and corr. Moscow, OAO "Meditsina" Publishing House. 2005., p.131-170.
8. I.V. Silkina, T.S. Gan'shina, S.B. Seredinin, P.S. Mirzoyan. Enhancement of Blood Supply to Ischemic Brain Under Effect of Afobazol. J. Exp. and Clin. Pharmacology, 2004, vol. 67, No 5, p.9-13.
9. Smith M.L., Bendek G., Dahlgen N. at all, Models for studing, long-term recovery following forebrain ischemia in the rat. A 2-vessell occlusion model.// Neurol.Scan., 1984, v.69, p.385 - 400.
10. Φ Y.Wang-Fisher. Manual of Stroke Models in rats. CRC press, 2008, p.3-4
11. Laboratory Animals Care and Keeping Program. Nursery for laboratory animals under Federal Institute of bioorganic chemistry, November 2005.

### INDUSTRIAL APPLICABILITY

The Invention is intended to be used in the manufacture of the medicinal drugs treatment of the central and peripheral nervous system diseases; it includes the manufacturing process.

## Claims

1. Method for producing a biologically active protein polypeptide complex formulation comprising actions involving quick frozen embryonic brain of hoof livestock of gestational age from the middle of the first trimester to the middle of the last trimester of pregnancy freezing and gradual, stage-by-stage, defrosting, at temperatures from 2°C to 28°C;
- homogenization in buffer solution with simultaneous extraction in the presence of proteolysis inhibitors and non-ionic detergents, including ethylenediamine tetraacetate, mannitol or maltose, with pH not less than 5.2 and not exceeding 8.5, with the volumetric solution-tissue ratio of at least 1 : 0.5;
- the homogenate is separated from undiluted tissue and cellular components by filtration and subsequent centrifugation, at "g" between 10000 and 30000, within 90-30 minutes, respectively;
- the supernatant is filtered through a membrane filter with a maximum pore size of 0.45 µm;
- the filtrate is subject to anion exchange chromatography at 2-4°C , with the buffer solution as the moving phase, separating proteins and polypeptides contacting with the adsorbent, with a step-by-step increment, using eluent with ionic strength ranging between 0.08 and 0.26 mol/l and pH - between 5.2 and 8.5, increasing the moving phase ionic strength by increment of 0.02 mol/l, starting to collect target fractions using the solution with ionic strength of at least 0.1; and
- collected target fractions are desalted using the method of dialysis or gel filtration and, after addition of conserving substances **characterized by** bacteriostatic and fungicide activity with the maximum total concentration of 0.06 mg/ml and solubilizator with the maximum total concentration of 0.01 mg/ml, ultrafiltration with the maximum pore size of 0.22 µm is effected, then sterile packaging is performed.

2. The biologically active protein polypeptide complex having tissue-specific restoration effect on neural tissues obtained from quick-frozen brain obtainable by the method described in claim 1, including negatively charged, mildly acidic, neutral proteins and polypeptides relating to the factors of growth, differentiation, signaling molecules determining its biological and pharmaceutical activity, with the molecular mass from 5 to 200 kDa, with at least 80% of the total protein mass having molecular mass ranging from 10 to 120 kDa, is characterized with a peak value at the wavelength of 274-284 nm in UV-visible spectrum and presence of bands in the pl range from 4.2 to 8.4 at isoelectric focusing in 5% polyacrylamide gel.

## Patentansprüche

1. Verfahren zur Herstellung eines biologisch aktiven Protein-Polypeptidkomplexes, unter Verwendung tiefgekühlten Hirns von behuften Nutzviehembryos des Gestationsalters von der Mitte des Ersttrimesters bis zur Mitte des Letzttrimesters der Schwangerschaft, umfassen das Einfrieren und das langsame, stufenweise Auftauen bei Temperaturen von 2°C bis 28°C. ;
- Homogenisierung in Pufferlösung unter gleichzeitiger Extraktion unter Verwendung von Proteolyseinhibitoren und nicht-ionischen Detergentien, umfassend Ethylendiamintetraacetat, Mannitol oder Maltose, mit einem pH von nicht weniger als 5.2 und nicht mehr als 8.5, mit einem Lösung-Gewebe-Verhältnis von mindestens 1 : 0,5;
- das Homogenisat wird vom nicht gelösten Geweben und von Zellkomponenten durch Filtration und anschließende Zentrifugation bei 10.000 g bis 30.000 g innerhalb von 90-30 Minuten, getrennt;
- der Überstand wird mit einem Membranfilter mit einer maximalen Porenweite von 0.45 µm filtriert;
- das Filtrat wird einer Anionenaustauscher-Chromatographie bei der Temperatur von 2-4°C mit der Pufferlösung als mobile Phase unterzogen, wobei Proteine und Polypeptiden durch Kontaktieren mit dem Absorber und mit einem schrittweisen Anwachs unter Verwendung eines Fließmittels mit einer Ionenstärke im Bereich von 0.08 bis 0.26 mol/l und einem pH im Bereich von 5.2 bis 8.5 getrennt werden, wobei die Ladungsstärke der mobilen Phase in Inkrementen von 0.02 mol/l anwächst und bei einer Ionenstärke von mindestens 0,1 begonnen wird die Zielfraktionen zu sammeln; und
- die gesammelten Zielfraktionen mit Hilfe von Dialyse oder Gelfiltration entsalzt werden, und nach der Zugabe von konservierenden Substanzen mit einer maximalen Konzentration von 0,06 mg/mL, die durch bakteriostatische und fungizide Eigenschaften gekennzeichnet sind und einem Solubilisator mit einer maximalen Gesamtkonzentration von 0,01 mg/mL anschließend eine Ultrafiltration bei einer maximalen Porenweite von 0.22 µm unterzogen werden und anschließend steril verpackt werden.

2. Der biologisch aktive Protein-Polypeptidkomplex mit spezifischer Erneuerungswirkung auf Nervengewebe erhältlich aus schockgefrorenem Embryohirn gemäß dem Verfahren nach Anspruch 1, umfassend negativ geladenen, leicht saure, neutrale Proteine und Polypeptide, die den Wachstums- und Differenzierungsfaktoren und Signalmolekülen zuzuordnen sind, die seine biologische und pharmazeutische Aktivität bestimmen, mit einer Molekülmasse von 5 bis 200 kDa, wobei min. 80% der Gesamtproteinmasse eine molare Masse von 10 bis 120 kDa haben, und durch einen Maximalwert bei einer Wellenlänge von 274-284 nm im UV-sichtbarem Spektrum und durch die Präsenz von Banden im pI Bereich von 4.2 bis 8.4 bei isoelektrischer Fokussierung im 5% Polyacrylamidgel gekennzeichnet sind.

## Revendications

1. Méthode d'obtention d'un groupe complexe proteino-polypeptidique biologiquement actif comprenant l'utilisation du cerveau congelé d'embryons de bétail domestique ongulé d'âge in utéro entre le milieu du premier trimestre et le milieu du dernier trimestre de la gestation, congelé et progressivement décongelé par températures de 2°C à 28°C;
- Homogénéisation dans une solution tampon avec extraction simultanée à l'aide d'inhibiteurs de protéolyse et de détersifs non ioniques, comprenant des étylène-diaminetétra-acétate, mannitol ou maltose, dont le pH n'est pas inférieur à 5,2 ni supérieur à 8,5, et dont le rapport solution-tissu n'est pas inférieur à 1 : 0,5;
- L'homogénat est séparé du tissu non dilué et des composants cellulaires par filtrage puis par centrifugation de 10000 à 30000 g en l'espace de 90-30 minutes, respectivement;
- Le surnageant est filtré avec un filtre membrane d'une grosseur de pores maximale de 0,45 microns;
- le filtrat subit une chromatographie à échange d'anions à température de 2-4°C, alors que la solution tampon est utilisée comme phase mobile, séparant les protéines et les polypeptides au contact avec un absorbant et avec une augmentation progressive par l'utilisation un agent éluant d'une force ionique entre 0,08 et 0,26 mol/l d'un pH entre 5,2 et 8,5, augmentant la force ionique de la phase mobile par un accroissement à 0,02 mol/l, et procédant à la collecte des fractions recherchées en utilisant la solution d'une force ionique d'au moins 0,1; et
- les fractions recherchées recueillies sont dessalées par le biais d'une dialyse ou d'un gel-filtrage et, après incorporation des substances conservatrices **caractérisées par** un effet bactériostatique et antifungique d'une concentration maximale à 0,06 mg/ml et d'un agent de solubilisation d'une concentration maximale à 0,01 mg/ml, subit une ultrafiltration par une grosseur maximal de pores de 0,22 microns et est ensuite emballée stérilement.

2. Le groupe complexe protéino-polypeptidique biologique actif exerçant un effet spécifique régénérateur sur les tissus nerveux obtenu du cerveau congelé d'embryons par la méthode selon la revendication 1, comprenant les protéines et polypeptides négatogènes neutres d'une faible acidité pour le facteur de croissance et de différenciation, les molécules de signalisation définissant son activité biologique et pharmaceutique, dont la masse moléculaire est entre 5 et 200 kDa, alors qu'au moins 80% de la masse globale protéique a une masse moléculaire entre 10 et 120 kDa, se **caractérise par** une valeur maximale de longueur d'onde de 274 à 284 nm au spectre ultraviolet visible et par la présence des bandes d'une étendue pl de 4,2 à 8,4 sous focalisation isoélectrique au gel polyacrylamide à 5%.
